# EUROPÄISCHE PATENTSCHRIFT

(11) **EP 4 294 907 B1**
(45) Veröffentlichungstag und Bekanntmachung des Hinweises auf die Patenterteilung: **15.04.2026**
(21) Anmeldenummer: 22728384.3
(22) Anmeldetag: 06.05.2022
(51) Int. Cl.: C12M 1/32, C12M 1/00

(54) **VORRICHTUNG UND VERFAHREN ZUR ZELLKULTIVIERUNG**
DEVICE AND METHOD FOR CELL CULTIVATION
DISPOSITIF ET PROCÉDÉ DE CULTURE CELLULAIRE

(30) Priorität: 07.05.2021 DE 102021204675
(43) Veröffentlichungstag der Anmeldung: 27.12.2023
(73) Patentinhaber: LPKF Laser & Electronics SE, 30827 Garbsen (DE)
(72) Erfinder: KRÜGER, Robin A., 30419 Hannover (DE); HEINE-DANK, Antonia, 30823 Garbsen (DE); OSTERMANN, Oktavia, 30419 Hannover (DE); RÖSENER, Bernd, 32457 Porta Westfalica (DE); SCHULZ-RUHTENBERG, Malte, 31515 Wunstorf (DE)
(74) Vertreter: Taruttis, Stefan Georg
(86) Internationale Anmeldenummer: PCT/EP2022/062301
(87) Internationale Veröffentlichungsnummer: WO 2022/234096

(56) Entgegenhaltungen:
- EP-A1- 1 867 612
- EP-A1- 1 867 612
- EP-A1- 2 011 857
- WO-A1-2016/041544
- WO-A1-2016/076795
- CN-A- 107 338 183
- DE-A1- 102019 201 350
- DE-U1- 202014 105 173
- US-A1- 2009 013 724

## Beschreibung

Die vorliegende Erfindung betrifft eine Vorrichtung, die Verwendung der Vorrichtung zur Zellkultivierung und ein Verfahren zur Zellkultivierung mit der Vorrichtung. Die Vorrichtung zeichnet sich dadurch aus, dass sie voneinander getrennte Näpfe zur Aufnahme von Zellen aufweist, die Näpfe in Kontakt mit einem größeren Volumen sind, so dass ein Flüssigkeitsaustausch stattfinden kann. Die Näpfe weisen ein großes Streckungsverhältnis von Tiefe zu Durchmesser auf, das Zellen in den Näpfen hält bzw. ein Austragen von Zellen aus den Näpfen in das mit den Näpfen in Kontakt stehende größere Volumen unterdrückt. Das Verfahren hat den Vorteil, Zellen, die vereinzelte oder mehrere Zellen sein können, in ihren Näpfen zu halten, wobei mehrere Näpfe mit einem gemeinsamen Volumen an Medium in Kontakt stehen. Durch das Halten von Zellen in Näpfen mit großem Streckungsverhältnis hat das Verfahren den Vorteil, dass Zellen anhand der sie enthaltenden Näpfe identifizierbar sind und über eine längere Kultivierungsdauer beobachtbar sind, z.B. über 1 bis 10d. Durch das große Streckungsverhältnis der Näpfe in Verbindung mit einer Variation der Diffusionsrate, z.B. durch Variation des Zu- und Ablaufs von Medium, können sich entweder für jeden Napf separat Stoffwechselprodukte darin sammeln und dem Napf und darin enthaltenen Zellen zugeordnet werden oder besonders konstante Bedingungen durch schnellen Austausch von Medium und Abführen von Stoffwechselprodukten geschaffen werden.

Konventionell werden für solche Verfahren sogenannte Mikrotiterplatten als Vorrichtungen zur Zellkultivierung und für die Durchführung biologischer Experimente eingesetzt. Mikrotiterplatten weisen Näpfe mit einem Volumen von z.B. ca. 300 µl auf. Pro Napf kann ein biologisches Experiment durchgeführt werden, pro Napf ist daher eine große Menge an Reagenzien notwendig. Zudem sind Mikrotiterplatten vergleichsweise groß und bieten nur Platz für typischerweise 96 und bis zu 1536 parallele Experimente. Einzelne Zellen in diesen Näpfen aufzufinden, z.B. mittels Lichtmikroskop, ist aufwändig.

Dies stellt Hochdurchsatzexperimente, bei denen bestimmte Zelltypen kultiviert und untersucht werden sollen, z.B. Zelllinien-Entwicklung, Entwicklung monoklonaler Antikörper, synthetische Biologie, Zelltherapie, Immuntherapie, Stammzellenforschung, vor große Herausforderungen.

Die Verwendung von stark miniaturisierten Näpfen, z.B. um den Faktor 100.000 verringerte Volumina, ist technisch möglich, bringt aber neue Herausforderungen mit sich, insbesondere Verdunstung, Zuführung von Nährstoffen, Aufkonzentrierung von Stoffwechselprodukten und Zellwanderung. Letzteres entsteht durch die Bewegung der Zellen aus eigener Kraft oder durch Turbulenzen beim Nachfüllen von Reagenzien oder Medien, beim Bewegen der Träger, durch Erwärmung und Konvektion.

Für die meisten biomedizinischen Entwicklungen müssen konstante Bedingungen in jedem einzelnen Experiment sichergestellt werden. Für die Zulassung medizinischer Produkte muss Monoklonalität nachgewiesen werden. Daher ist die Aufgabe dieser Erfindung, die o.g. Herausforderungen zu lösen.

### Stand der Technik

Die DE 10 2020 209 825 A1, die nach dem Prioritätstag der vorliegenden Anmeldung veröffentlicht ist und daher nur in Bezug auf die Neuheit Beachtung finden kann, beschreibt ein Verfahren zur Herstellung eines Kunststoffteils, das einen Glaseinsatz aufweist, aus einem vorgefertigten Kunststoffteil mit einem umlaufenden Schweißsteg und einem Glaseinsatzes, der in einem umfänglich geschlossenen Anlagebereich beabstandet angeordnete Durchgangslöcher hat und Erwärmen des Schweißstegs und Pressen des Glaseinsatzes mit seinem Anlagebereich gegen den Schweißsteg des Kunststoffteils zur Herstellung eines Verbindungsbereichs, in dem Kunststoff im Anlagebereich aufliegt und durch die Durchgangslöcher des Glaseinsatzes hindurchgetreten ist.

Die DE 10 2019 217 466 A1, die nach Art. 54 (3) EPÜ nur in Bezug auf die Neuheit Beachtung finden kann, beschreibt Reaktionsgefäße aus Glas, die als Ausnehmungen von zumindest 30 µm Tiefe in einer einstückigen Glasplatte oder in zwei miteinander verbundenen Glasplatten gebildet sind. Die Ausnehmungen können einen Abschnitt aufweisen, der sich nur bis in einen ersten Dickenabschnitt in die Glasplatte erstreckt, von dem aus sich weitere kleinere Ausnehmungen bis in einen tieferen zweiten Dickenabschnitt erstrecken können.

Die WO 2016/041544 A1 beschreibt generell ein für die Herstellung von Ausnehmungen in Glas in der vorliegenden Erfindung bevorzugtes Verfahren.

Die WO 2016/076795 A1 beschreibt eine Mikrofluidikplatte aus Kunststoff mit umfänglich geschlossenen Kanälen zur Zellkultivierung, mit an jedem Ende einem Anschluss für eine Zu- oder Ableitung.

Die CN 107338183 A zeigt einen Träger mit für Zellen geeigneten Ausnehmungen, die jeweils im Boden eine Bohrung zur Verbindung mit einer der Ausnehmung gegenüberliegenden Auslassleitung haben, wobei die Ausnehmungen von einem beabstandeten Deckel überdeckt sind, der eine gemeinsame Zuleitung einfasst.

Die DE 20 2014 105 173 U1 zeigt zum Abdecken der Näpfe einer Mikrotiterplatte einen beidseitig offenen Rahmen, der vom vorstehenden Rand einer Deckelplatte überdeckt und umfasst wird, um eine Seite des Rahmens zu überdecken, wenn ein Mikrotiterplatte in den Rahmen eingeklemmt ist.

Die EP 2 011 857 A1 beschreibt die Herstellung eines Behälters für die Zellkultur mit Ausnehmungen, deren Böden noch kleinere Vertiefungen haben

### Aufgabe der Erfindung

Der Erfindung stellt sich die Aufgabe, ein alternatives Verfahren zur Kultivierung von Zellen und einen zur Verwendung im Verfahren geeigneten Träger mit Ausnehmungen für Zellen bereitzustellen, um Zellen in ihren separaten Näpfen zu halten und dennoch mehrere Näpfe mit einem gemeinsamen Volumen an Medium zu kontaktieren, das einen gemeinsamen Vorrat an Medium bildet. Dabei soll der Träger eingerichtet sein, Flüssigkeit, insbesondere Zellkulturmedium, gezielt aus Ausnehmungen ablaufen zu lassen bzw. zu entnehmen.

### Beschreibung der Erfindung

Die Kultivierung von Zellen ist vorliegend ein in vitro-Verfahren, insbesondere die Kultivierung von Zellen in Medium, das die Zellen lebend erhält und/oder zur Zellvermehrung geeignet ist. Die Zellen sind z.B. tierische, insbesondere menschliche Zellen, pflanzliche oder Pilz- bzw. Hefezellen oder Bakterien. Die Kultivierung kann z.B. zur Analyse und/oder Isolierung von Zellen erfolgen, optional mit Zusetzen eines Wirkstoffs zum Medium.

Die Erfindung löst die Aufgabe mit den Merkmalen der Ansprüche und insbesondere durch einen Träger, der zur Kultivierung von Zellen geeignet ist und bevorzugt ein plattenförmiger Träger, z.B. aus Glas, Silizium oder Kunststoff oder einer Kombination davon, ist, in dem zumindest eine erste Ausnehmung gebildet ist, die sich über einen ersten Dickenabschnitt des Trägers erstreckt, wobei sich von der ersten Ausnehmung eine Anordnung von zweiten Ausnehmungen in einen an den ersten Dickenabschnitt angrenzenden zweiten Dickenabschnitt des Trägers erstreckt. Der erste Dickenabschnitt kann eine Dicke von wenigen Mikrometern bis einigen Zentimetern aufweisen. Die zweiten Ausnehmungen, die sich ausgehend von einer ersten Ausnehmung in den zweiten Dickenabschnitt erstrecken und jeweils eine zweite Anordnung bilden, bilden Näpfe, die zur Aufnahme von Zellen und/oder synthetischen Partikeln, z.B. aus Kunststoff oder Glas, geeignet sind. Die zweiten Ausnehmungen, auch als Näpfe bezeichnet, haben ein großes Streckungsverhältnis von Tiefe zu Durchmesser, der in der Ebene des ersten Dickenabschnitts angrenzend an den zweiten Dickenabschnitt oder auf der halben Höhe des zweiten Dickenabschnitts bestimmt wird. Die Näpfe haben z.B. ein Streckungsverhältnis von Tiefe zu Durchmesser von mindestens 1:1, bevorzugt mindestens 2: 1, bevorzugt mindestens 5:1, bevorzugt mindestens 10:1, bevorzugt mindestens 20:1, bevorzugt mindestens 40:1, bevorzugt mindestens 50:1, wobei die Näpfe einen Durchmesser von maximal 1000 µm, bevorzugt maximal 900 µm, bevorzugt maximal 800 µm, bevorzugt maximal 700 µm, bevorzugt maximal 600 µm, bevorzugt maximal 500 µm, bevorzugt maximal 400 µm, bevorzugt maximal 300 µm, bevorzugt maximal 200 µm, bevorzugt maximal 100 µm, bevorzugt maximal 90 µm, bevorzugt maximal 80 µm, bevorzugt maximal 70 µm, bevorzugt maximal 60 µm, bevorzugt maximal 50 µm, bevorzugt maximal 40 µm, bevorzugt maximal 30 µm, bevorzugt maximal 20 µm, bevorzugt maximal 10 µm, bevorzugt maximal 5 µm aufweisen.

Der Durchmesser der Näpfe kann an die Größe der Zellen angepasst werden, beispielsweise um Zellen in einem Stapel anordnen zu können oder in Berührung zueinander zu bringen. Der Querschnitt der einzelnen Näpfe kann rechteckig, A-förmig, V-förmig oder tailliert sein. Der Boden der Näpfe kann flach, spitz zulaufend (konisch) oder abgerundet sein. Der Boden kann weitere Mikrostrukturen aufweisen.

Der erste Dickenabschnitt wird in einer Ebene durch die erste Oberfläche des Trägers begrenzt, in der die Querschnittsöffnungen der ersten Ausnehmungen liegen, und in einer davon beabstandeten Ebene von den Querschnittsöffnungen der zweiten Ausnehmungen. Die Querschnittsöffnungen der zweiten Ausnehmungen sind durch Oberflächenbereiche des Trägers, der insbesondere ein zweiter Teilträger ist, beabstandet. Weiter bevorzugt liegen diese Oberflächenbereiche und die Querschnittsöffnungen der zweiten Ausnehmungen in einer gemeinsamen Ebene. Erfindungsgemäß grenzen die Querschnittsöffnungen der zweiten Ausnehmungen unmittelbar an den Querschnitt einer ersten Ausnehmung. -

Die Anordnung der zweiten Ausnehmungen kann beliebig sein, ist aber bevorzugt regelmäßig, z.B. in parallelen Reihen, in Knotenpunkten eines Gitters und/oder in jeweils gleichen Abständen, das z.B. ein quadratisches oder hexagonales Gitter ist, angeordnet. Eine Anordnung der zweiten Ausnehmungen, die sich angrenzend an eine erste Anordnung erstreckt, kann z.B. mindestens 90, bevorzugt mindestens 100, bevorzugt mindestens 200, bevorzugt mindestens 300, bevorzugt mindestens 400, bevorzugt mindestens 500, bevorzugt mindestens 600, bevorzugt mindestens 700, bevorzugt mindestens 800, bevorzugt mindestens 900, bevorzugt mindestens 1000, bevorzugt mindestens 2000, bevorzugt mindestens 3000, bevorzugt mindestens 4000, bevorzugt mindestens 5000, bevorzugt mindestens 6000, bevorzugt mindestens 7000, bevorzugt mindestens 8000, bevorzugt mindestens 9000, bevorzugt mindestens 10.000, bevorzugt mindestens 50.000, bevorzugt mindestens 100.000, bevorzugt mindestens 500.000, bevorzugt mindestens 1.000.000, bevorzugt mindestens 10.000.000, bevorzugt mindestens 50.000.000, bevorzugt mindestens 100.000.000 zweite Ausnehmungen aufweisen, die in einem Abstand von maximal 10 mm, bevorzugt maximal 9 mm, bevorzugt maximal 8 mm, bevorzugt maximal 7 mm, bevorzugt maximal 6 mm, bevorzugt maximal 5 mm, bevorzugt maximal 4 mm, bevorzugt maximal 3 mm, bevorzugt maximal 2 mm, bevorzugt maximal 1 mm, bevorzugt maximal 900 µm, bevorzugt maximal 800 µm, bevorzugt maximal 700 µm, bevorzugt maximal 600 µm, bevorzugt maximal 500 µm, bevorzugt maximal 400 µm, bevorzugt maximal 300 µm, bevorzugt maximal 200 µm, bevorzugt maximal 100 µm, bevorzugt maximal 90 µm, bevorzugt maximal 80 µm, bevorzugt maximal 70 µm, bevorzugt maximal 60 µm, bevorzugt maximal 50 µm, bevorzugt maximal 40 µm, bevorzugt maximal 30 µm, bevorzugt maximal 20 µm, bevorzugt maximal 10 µm, bevorzugt maximal 9 µm, bevorzugt maximal 8 µm, bevorzugt maximal 7 µm, bevorzugt maximal 6 µm, bevorzugt maximal 5 µm, bevorzugt maximal 4 µm, bevorzugt maximal 3 µm, bevorzugt maximal 2 µm, bevorzugt maximal 1 µm voneinander angeordnet sind.

Die Querschnittsöffnung der ersten Ausnehmung liegt in der Ebene einer ersten Oberfläche des Trägers, so dass die erste Ausnehmung in der Ebene der ersten Oberfläche des Trägers mündet. Der Träger zeichnet sich durch einen Auslass aus, dessen Querschnitt im oder angrenzend an den ersten Dickenabschnitt mündet, wobei der Querschnitt des Auslasses bevorzugt an die Ebene der ersten Oberfläche des Trägers angrenzt, bzw. der zur Ebene der ersten Oberfläche des Trägers offen ist, wobei der Auslass mit der ersten Ausnehmung verbunden ist. Der Auslass ist im Träger ausgebildet, z.B. in Form eines Grabens, der zur Ebene der ersten Oberfläche des Trägers offen ist. Alternativ kann der Auslass eine Leitung sein, die sich in die erste Ausnehmung erstreckt. Generell ist der Auslass eingerichtet, Flüssigkeit, insbesondere Medium, aus der ersten Ausnehmung auszutragen. Dabei kann der Auslass eingerichtet sein, Flüssigkeit allein durch die Wirkung der Schwerkraft und/oder mittels einer Pumpe und/oder durch Beaufschlagen der ersten Ausnehmung mit Überdruck auszutragen. Der Auslass kann einen Querschnitt aufweisen, der sich in den Träger in eine Tiefe erstreckt, die ein Anteil von z.B. 1 bis 95 % des ersten Dickenabschnitts ist. Ein solcher Auslass dient zum Ablaufenlassen oder Entnehmen von Flüssigkeit, die sich in der ersten Ausnehmung bis in den Querschnitt des Auslasses erstreckt, insbesondere bei einem Pegelstand von Flüssigkeit in der ersten Ausnehmung bis an die Ebene der ersten Oberfläche des Trägers. Der Auslass kann sich umfänglich um zumindest eine, bevorzugt genau eine erste Ausnehmung erstrecken, so dass der Auslass eine umfängliche Ausnehmung im Träger um eine erste Ausnehmung bildet, wobei die umfängliche Ausnehmung vom Träger begrenzt wird, der sich im Abstand dieses Auslasses bis in die Ebene seiner ersten Oberfläche erstreckt. In der Ausführungsform des Auslasses als umfänglich um eine erste Ausnehmung ausgebildete Ausnehmung oder hydrophile Oberfläche, können genau eine oder zumindest zwei Verbindungsleitungen mit diesem Auslass verbunden sein. Optional kann der Auslass zusätzlich einen mit der ersten Ausnehmung verbundenen Einlass bilden, insbesondere für Flüssigkeit, z.B. Medium, oder Gas.

Die Rate, mit der Flüssigkeit am Auslass austritt bzw. entnommen wird, kann durch die Dicke des ersten Dickenabschnitts, durch die Rate der Zuführung von Flüssigkeit oder durch Neigung des Trägers gesteuert werden. Der Auslass kann durch mehrere Öffnungen ausgeführt werden, beispielsweise Löcher mit verschiedenen Durchmessern. Ablaufende Flüssigkeit kann gesammelt und analysiert oder weiterverwendet werden.

In einer weiteren Ausführungsform kann der Träger als Auslass eine hydrophile Oberfläche aufweisen, die jeweils genau eine oder zumindest zwei erste Ausnehmungen umfasst, wobei dieser Auslass von einer hydrophoben Oberfläche bzw. Beschichtung der ersten Oberfläche des Trägers eingefasst ist. In dieser Ausführungsform kann die als Auslass eine erste Ausnehmung umfassende hydrophile Oberfläche in derselben Ebene liegen, wie die erste Oberfläche des Trägers, der bevorzugt angrenzend hydrophob ist. Auch in dieser Ausführungsform kann eine Verbindungsleitung in dem Auslass angeordnet sein, der aus einem hydrophilen Oberflächenabschnitt auf dem Träger bestehend, optional ohne Ausnehmung im Träger, ausgebildet ist. Im Verfahren erlaubt die hydrophile Oberfläche dieses Auslasses, auch wenn er in der Ebene der ersten Oberfläche des Trägers liegt, den Übertritt von Flüssigkeit aus der ersten Ausnehmung in die Verbindungsleitung, wobei die Ausbreitung der Flüssigkeit auf die erste Oberfläche durch die an diesen Auslass angrenzende Hydrophobie der ersten Oberfläche begrenzt wird.

Der Träger ist durch den Auslass und bevorzugt durch eine mit dem Auslass verbundene Verbindungsleitung für ein Verfahren zur Zellkultivierung eingerichtet, bei dem Flüssigkeit, insbesondere Zellkulturmedium, gezielt aus Ausnehmungen ablaufen bzw. entnommen werden kann. Eine Verbindungsleitung verbindet den Auslass mit einer davon entfernten Abnahmestelle, die z.B. ein Behälter für Flüssigkeit oder eine Analyseeinrichtung sein kann. Generell kann eine Verbindungsleitung als Ausnehmung im Träger ausgebildet sein, die einen zur Oberfläche des Trägers offenen Querschnitt hat, wie z.B. ein Graben, oder die einen umfänglich geschlossenen Querschnitt hat, wie z.B. eine durch den Träger gehende Bohrung.

In einer Ausführungsform ist innerhalb des Trägers und/oder an einer Stirnfläche des Trägers eine Verbindungsleitung ausgebildet, die sich von der ersten Oberfläche des Trägers über dessen gesamten Querschnitt bis auf dessen zweite Oberfläche erstreckt. Die Verbindungsleitung ist gegenüber der zweiten Oberfläche des Trägers mit dem Auslass verbunden, so dass Flüssigkeit aus dem Auslass durch die Verbindungsleitung in die Ebene der zweiten Oberfläche des Trägers fließen kann, insbesondere bei horizontaler Anordnung des Trägers mit dessen erster Oberfläche oberhalb der zweiten Oberfläche.

In Ausführungsformen leitet die Verbindungsleitung Flüssigkeit, deren Pegelstand in der ersten Ausnehmung den Querschnitt des Auslasses erreicht und durch den Auslass fließt, auf die gegenüberliegende zweite Oberfläche des Trägers. Bei Zuführen von Flüssigkeit in die erste Ausnehmung wird daher Flüssigkeit, deren Pegelstand den Querschnitt des Auslasses erreicht, kontrolliert abgeleitet und der Pegelstand wird den Querschnitt des Auslasses und insbesondere die erste Oberfläche des Trägers nicht überschreiten. Die Mündung der Verbindungsleitung in der zweiten Oberfläche des Trägers erlaubt das Abnehmen dort austretender Flüssigkeit, z.B. zur anschließenden Analyse, ohne dass eine zusätzliche Entnahmevorrichtung, z.B. eine Pipette, mit der Flüssigkeit in der ersten Ausnehmung in Kontakt tritt.

Die Oberflächen der Verbindungsleitung und des Auslasses sind bevorzugt hydrophil, weiter bevorzugt ist die erste Oberfläche des Trägers und/oder die gegenüberliegende zweite Oberfläche des Trägers hydrophob, z.B. mit einer hydrophoben Beschichtung versehen. Optional ist in der zweiten Oberfläche des Trägers in einem Abstand um die Mündung der Verbindungsleitung eine Nut ausgebildet, die die Mündung einfasst. Eine in der zweiten Oberfläche des Trägers die Mündung der Verbindungsleitung einfassende Nut verringert die Ausbreitung von Flüssigkeit, die aus der Mündung austritt, über die zweite Oberfläche des Trägers.

Bevorzugt weist ein Träger zumindest zwei erste Ausnehmungen auf, an deren ersten Dickenabschnitt sich jeweils eine Anordnung zweiter Ausnehmungen anschließt, wobei jede erste Anordnung mittels zumindest eines Auslasses mit zumindest einer oder genau einer Verbindungsleitung verbunden ist. In dieser Ausführungsform sind zumindest zwei erste Ausnehmungen unabhängig voneinander mit jeweils einer Verbindungsleitung verbunden, so dass aus der ersten Ausnehmung austretende Flüssigkeit jeweils separat für jede erste Ausnehmung auf der zweiten Oberfläche aus einer Verbindungsleitung austritt und separat abgenommen und/oder analysiert werden kann.

Optional weist ein Träger zumindest zwei erste Ausnehmungen auf, an deren ersten Dickenabschnitt sich jeweils eine Anordnung zweiter Ausnehmungen anschließt, wobei der mit der einen ersten Ausnehmung verbundene Auslass in einer anderen ersten Ausnehmung mündet, so dass der Auslass eingerichtet ist, aus der einen ersten Ausnehmung austretende Flüssigkeit in die andere erste Ausnehmung zu leiten.

Generell kann optional angrenzend an die erste Oberfläche des Trägers zumindest eine Zuleitung im Träger ausgebildet sein, die in zumindest einer ersten Ausnehmung mündet. Eine Zuleitung kann z.B. als Graben im Träger ausgebildet sein, wobei der Querschnitt des Grabens in der Ebene der ersten Oberfläche des Trägers offen ist. Dabei kann in einem Abstand von einer ersten Ausnehmung eine sich in den Träger erstreckende Vorratsausnehmung mit der Zuleitung verbunden sein. Eine Vorratsausnehmung, z.B. mit einer Tiefe gleich oder geringer als die Tiefe der Zuleitung, kann zum Aufnehmen von Flüssigkeit, z.B. Zellkulturmedium, dienen, die als Vorrat in die Vorratsausnehmung dosiert wird.

Die ersten Ausnehmungen und die zweiten Ausnehmungen können in einem einstückigen Träger, bevorzugt einem Glasträger, gebildet sein. Ausnehmungen in einem einstückigen Träger können durch Entfernen von Material aus einem ursprünglichen einstückigen Träger hergestellt werden, z.B. durch Laserbestrahlen und anschließendes Ätzen. Alternativ können die ersten Ausnehmungen Durchgangslöcher in einem ersten Teilträger sein, an dem ein zweiter Teilträger angeordnet ist, in dem die zweiten Ausnehmungen ausgebildet sind. Bei Ausbildung des Trägers aus zumindest zwei Teilträgern ist der Träger zweistückig, wobei die Teilträger direkt miteinander verbunden sein können, z.B. durch Bonden, oder durch eine Verbindungsmasse, z.B. geschmolzene Glasfritte oder Klebstoff, miteinander verbunden sein können. Die zweiten Ausnehmungen können Durchgangslöcher in dem zweiten Teilträger sein, deren Querschnittsöffnung gegenüber dem ersten Teilträger von einem dritten Teilträger überdeckt sind, oder Sacklöcher in dem zweiten Teilträger. Optional ist der zweite Teilträger aus gefärbtem Glas, der optionale dritte Teilträger aus transparentem Glas, und der erste Teilträger ist aus ungefärbtem bzw. transparentem Glas. Dabei ist transparentes Glas für Licht durchlässig, das zur optischen Detektion auf den Träger eingestrahlt wird und für Licht durchlässig, das zur optischen Detektion von zweiten Ausnehmungen ausgehen kann. Gefärbtes Glas ist bevorzugt für Licht undurchlässig, das zur optischen Detektion auf den Träger eingestrahlt wird oder für Licht undurchlässig, das zur optischen Detektion von zweiten Ausnehmungen ausgehen kann. Bevorzugt ist der Träger bzw. sind alle Teilträger, aus Glas.

Das große Streckungsverhältnis der zweiten Ausnehmungen führt dazu, dass darin eingebrachte Zellen in einer zweiten Ausnehmung bleiben und erlaubt ein Verfahren zur Zellkultivierung, bei dem Flüssigkeit in die erste Ausnehmung eingebracht oder daraus austritt, während die Zellen in den zweiten Ausnehmungen bleiben. Dabei stehen die Innenvolumina der zweiten Ausnehmungen mit dem Innenvolumen der ersten Ausnehmung in Kontakt, so dass ein Austausch zwischen diesen erfolgt, z.B. durch Diffusion von Stoffwechselprodukten aus zweiten Ausnehmungen in die erste Ausnehmung und von Nährstoffen, gelöstem Sauerstoff und ggf. zugesetzten Wirkstoffen aus der ersten Ausnehmung in zweite Ausnehmungen.

Optional ist ein Sensor in einer ersten Ausnehmung, in einer der zweiten Ausnehmungen, im Auslass und/oder im Einlass angeordnet, z.B. in einer Verbindungsleitung. Ein Sensor kann ein Gassensor sein, z.B. für Sauerstoff oder Kohlendioxid, ein pH-Sensor, Temperatursensor, oder ein für einen Analyten spezifisches Bindemolekül, vorzugsweise immobilisiert sein, z.B. ein Antikörper, der für einen Analyten im Medium spezifisch ist. Weiter optional kann im Einlass und im Auslass jeweils ein gleicher Sensor angeordnet sein, die mit einer Auswerteeinheit verbunden sind, die eingerichtet ist, Änderungen der Sensorsignale festzustellen.

Das Einbringen von Flüssigkeit in erste Ausnehmungen kann durch Fallenlassen von Tropfen oder Einfüllen durch eine Leitung, die in Kontakt mit der Flüssigkeit in der ersten Ausnehmung steht, jeweils durch die Querschnittsöffnung der ersten Ausnehmung in der Ebene der ersten Oberfläche des Trägers erfolgen. Alternativ kann der Träger eine Zuleitung aufweisen, die sich von dessen zweiter Oberfläche direkt in die erste Ausnehmung erstreckt, so dass die Zuleitung eingerichtet ist, Flüssigkeit und/oder Gas von der zweiten Oberfläche in die erste Ausnehmung zu leiten. Generell ist bei horizontaler Anordnung des Trägers dessen zweite Oberfläche horizontal und unterhalb seiner ersten Oberfläche angeordnet, wobei bevorzugt die erste und zweite Oberfläche parallel zueinander liegen. Alternativ oder zusätzlich kann sich eine Zuleitung durch einen Wandabschnitt innerhalb des ersten Dickenabschnitts erstrecken, in dem eine erste Ausnehmung ausgebildet ist, so dass durch die Zuleitung Flüssigkeit, z.B. Medium für die Zellkultur, im ersten Dickenabschnitt in die erste Ausnehmung eingeführt werden kann.

Im Träger kann im ersten Dickenabschnitt ein Auslass als Leitung ausgebildet sein, z.B. in einer ersten Ausnehmung in einer Wand, die einer Zuleitung gegenüber liegt.

In einer Ausführungsform kann die Wand, die sich über den ersten Dickenabschnitt erstreckt und die ersten Ausnehmungen aufweist bzw. diese umfasst, von einem ersten Teilträger gebildet sein, der an einen zweiten Teilträger angrenzt, in dem zweite Ausnehmungen gebildet sind. Dabei kann der erste Teilträger auf den zweiten Teilträger aufgesetzt werden, z.B. dadurch, dass sich der erste Teilträger und der zweite Teilträger im Randbereich verschieblich und flüssigkeitsdicht überlappen. Optional kann der erste Teilträger flüssigkeitsdicht senkrecht zum zweiten Teilträger verschieblich sein, so dass durch die Verschieblichkeit des ersten Teilträgers relativ zum zweiten Teilträger der erste Dickenabschnitt einstellbar ist. Bei Ausbildung erster Ausnehmungen in einem ersten Teilträger, der flüssigkeitsdicht und aufsetzbar bzw. verschieblich zum zweiten Teilträger angeordnet ist, in dem zweite Ausnehmungen gebildet sind, ist der Träger eingerichtet, den Pegel der Flüssigkeit in der ersten Ausnehmung abhängig von der Stellung des ersten Teilträgers zum zweiten Teilträger einzustellen. Denn der Auslass, insbesondere wenn er kein Ventil aufweist, führt Flüssigkeit aus der ersten Ausnehmung ab, deren Pegel seinen Querschnitt erreicht. Dabei kann der erste Teilträger aus Kunststoff bestehen und der zweite Teilträger aus Glas, oder der erste und der zweite Teilträger können aus Glas bestehen, optional mit einer verschieblichen Dichtung im Überlappungsbereich der Teilträger. Alternativ kann zur Einstellung des Flüssigkeitspegels in der ersten Ausnehmung ein Ventil im Einlass und/oder im Auslass angeordnet sein, mit dem z.B. im Verfahren der Flüssigkeitspegel in der ersten Ausnehmung eingestellt werden kann.

In einer Ausführungsform können Einlässe und/oder Auslässe ein oder mehrere Ventile aufweisen, die eine oder mehrere Verbindungsleitungen regulieren. Mit mehreren Ventilen und mehreren Verbindungsleitungen ist es möglich, unterschiedliche Flüssigkeiten gesteuert in die erste Ausnehmung zu dosieren.

In einer Ausführungsform kann der Träger aus einem zweiten Teilträger mit darin angeordneten zweiten Ausnehmungen und einem daran angebrachten ersten Teilträger bestehen, der genau eine erste Ausnehmung aufweist, die sich über alle zweiten Ausnehmungen erstreckt. Generell und insbesondere in dieser Ausführungsform kann der erste Teilträger aus Kunststoff oder Glas bestehen und der zweite Teilträger aus Glas mit zweiten Ausnehmungen darin. In Ausführungsformen, in denen der erste Teilträger aus Kunststoff besteht und der zweite Teilträger aus Glas, ist bevorzugt, dass der zweite Teilträger im Bereich der Verbindung mit dem ersten Teilträger Durchgangslöcher aufweist und sich Kunststoff des ersten Teilträgers einstückig durch diese Durchgangslöcher erstreckt, z.B. dadurch, dass Kunststoff des ersten Teilträgers nach Erwärmen von erstem und/oder zweitem Teilträger im Verbindungsbereich in und ggf. durch diese Durchgangslöcher gedrückt ist. Bevorzugt weist der erste Teilträger in einem Abstand von dem Bereich, den der zweite Teilträger überdeckt, einen Auslass auf, optional mit daran angeschlossener Verbindungsleitung.

Ausnehmungen in Trägern aus Glas werden bevorzugt durch Bestrahlen des Glases mit Laserstrahlung und anschließendem Ätzen hergestellt.

Generell kann in jeder Ausführungsform eine erste Ausnehmung durch eine Membran unterteilt werde, die bevorzugt senkrecht zur ersten Oberfläche des Trägers angeordnet ist, so dass sie z.B. bei horizontaler Anordnung des Trägers bzw. dessen erster Oberfläche vertikal angeordnet ist. Eine Membran, die eine erste Ausnehmung unterteilt, kann z.B. durch einen Rahmen aufgespannt werden, der z.B. an Oberflächen des Trägers anliegt, die zwischen den Querschnittsöffnungen der zweiten Ausnehmungen liegen.

Im Verfahren wird generell Flüssigkeit in die erste Ausnehmung eingebracht, während in den zweiten Ausnehmungen Flüssigkeit mit zumindest einer Zelle enthalten ist. Dies wird z.B. erreicht, indem Flüssigkeit mit Zellen gezielt in zweite Ausnehmungen eingebracht wird, z.B. mittels eines Tropfenerzeugers bzw. einer Dosiereinrichtung, der insbesondere eine Düse ist. Alternativ kann eine Zellen enthaltende Flüssigkeit in die erste Ausnehmung eingebracht werden, so dass sie sich in die zweiten Ausnehmungen verteilt. So können sich z.B. Zellen aus der Flüssigkeit in die zweiten Ausnehmungen verteilen. Optional kann die Zellen enthaltende Flüssigkeit, die in der ersten Ausnehmung verbleibt, anschließend entfernt werden. Dabei kann die Flüssigkeit, die bevorzugt ein Zellkulturmedium ist, in Mischung mit darin suspendierten Zellen vorliegen und als Mischung bzw. Suspension in die Ausnehmungen eingebracht werden, oder die Flüssigkeit, die die Zellen enthält, kann in einem separaten Schritt und mittels eines separaten Tropfenerzeugers bzw. einer separaten Dosiereinrichtung in Ausnehmungen eingebracht werden und ein Zellkulturmedium, das keine Zellen enthält, kann in einem separaten Schritt, bevorzugt anschließend, in Ausnehmungen eingebracht werden, bevorzugt mittels einer separaten Dosiereinrichtung.

Vorliegend umfasst ein Zellkulturmedium ein Medium, das Zellen, insbesondere Pflanzenzellen, Hefen, Bakterien oder tierische Zellen, bevorzugt menschliche Zellen, lebensfähig erhält, z.B. für zumindest 1h, bevorzugt für zumindest 12h oder zumindest 24h oder zumindest zwei Tage oder zumindest drei Tage oder zumindest vier Tage oder zumindest fünf Tage oder zumindest sechs Tage oder zumindest sieben Tage.

In einer Ausführungsform des Verfahrens, bei der der Träger optional nur Ausnehmungen in Form der zweiten Ausnehmungen aufweist und weiter optional zumindest einen Auslass, optional zumindest eine damit verbundene Verbindungsleitung, oder daraus besteht, kann Flüssigkeit unmittelbar auf die Querschnittsöffnungen der zweiten Ausnehmungen, die in der Ebene der ersten Oberfläche des Trägers liegen, aufgebracht werden. In einer Ausführungsform, in der der Träger nur zweite Ausnehmungen aufweist und Medium über die Ebene der Querschnittsöffnungen der zweiten Ausnehmungen ragt, bildet diese überstehende Schicht aus Medium das Volumen der ersten Ausnehmung und erstreckt sich über den ersten Dickenabschnitt, so dass dieses Medium die zweiten Ausnehmungen überdeckt. Dabei kann sich das Medium über einen ersten Dickenabschnitt von z.B. 1 µm bis 3 mm, z.B. 10 µm bis 200 µm oberhalb der zweiten Ausnehmungen erstrecken. Die über die Ebene der ersten Oberfläche des Trägers ragende Flüssigkeit kann über den Rand des Trägers ablaufen, bevorzugt entlang des zumindest einen Auslasses und optional entlang der Verbindungsleitung. Dabei kann diese Flüssigkeit mittels Neigen des Trägers, durch Beaufschlagen mit einem Gasstrom und/oder Vibration, z.B. Ultraschall, zu Bewegung angeregt werden. Insbesondere in dieser Ausführungsform kann die erste Oberfläche für wässrige Flüssigkeiten schlecht benetzbar sein, z.B. hydrophob sein, z.B. aus Kunststoff oder einer strukturierten, funktionalisierten oder beschichteten Glasoberfläche. Auf die erste Oberfläche bzw. auf die Ebene, in der die Querschnittsöffnungen der zweiten Ausnehmungen liegen, aufgebrachter Flüssigkeitstropfen kann bei Bewegung über diese Ebene einen Stoffaustausch mit dort vorhandener Flüssigkeit erfahren. Z.B. kann ein auf diese Ebene aufgebrachter Tropfen aus Medium als rollender Tropfen bereits vorhandene Flüssigkeit, z.B. verbrauchtes Medium, aufnehmen und/oder teilweise ersetzen. Optional kann die Oberfläche des Trägers, die in der Ebene ist, in der die Querschnittsöffnungen der zweiten Ausnehmungen liegen, in den Träger eingebrachte Kanäle aufweisen, deren Querschnitt zu der Ebene offen ist, um die sich über die Ebene erstreckende Flüssigkeit bei deren Bewegung zu lenken. Dabei kann die Flüssigkeit stoßweise oder satzweise als zumindest zwei Tropfen auf diese Ebene des Trägers aufgebracht werden. Bei Überschreiten des Volumens an Flüssigkeit oberhalb der Ebene, in der sich der Träger erstreckt, wird Flüssigkeit ablaufen, insbesondere gezielt durch einen Auslass.

Generell verhindert Flüssigkeit, bevorzugt Medium, das sich über den ersten Dickenabschnitt erstreckt, die Verdunstung von Medium aus den zweiten Ausnehmungen. Der erste Dickenabschnitt kann dabei wenige Mikrometer bis einige Zentimeter groß sein. Das große Volumen der ersten Dickenabschnitts bietet dabei Platz für ausreichend Flüssigkeit, um die Effekte von Nährstoffverbrauch und Anreicherung von Stoffwechselprodukten in den zweiten Ausnehmungen durch Diffusion zu verringern. Das große Streckungsverhältnis der zweiten Ausnehmungen hat die Wirkung, dass Bewegungen der Flüssigkeit im angrenzenden ersten Dickenabschnitt bzw. der ersten Ausnehmung die Zellen in den zweiten Ausnehmungen nur unwesentlich bewegt und die Zellen nicht aus den zweiten Ausnehmungen heraus gespült werden.

Beispielsweise kann eine erste Ausnehmung mit 100000 zweiten Ausnehmungen verbunden sein, wobei jede zweite Ausnehmung einen Durchmesser von 54 µm, eine Tiefe von 436 µm, ein Streckungsverhältnis Tiefe zu Durchmesser von 8:1 und ein Volumen von 1 nl aufweist. Die Ausnehmungen weisen zueinander beispielsweise einen Abstand von 10 µm auf. Um alle zweiten Ausnehmungen zu umfassen, muss die erste Ausnehmung eine Fläche von mindestens 410 mm² aufweisen. Konventionell weisen Näpfe deutlich kleinere Streckungsverhältnisse auf, beispielsweise 0,16:1. Bei gleichem Volumen von 1 nl pro Napf und einem Durchmesser von 200 µm beträgt die Tiefe ca. 32 µm. In diesem Fall nehmen 100,000 Ausnehmungen eine Fläche von 4410 mm² ein, zehnmal so groß, wie die Fläche der Näpfe mit großem Streckungsverhältnis.

Durch die geringere Fläche bei gleichem Volumen pro Napf ist es möglich, mit einem optischen Mikroskop die Inhalte mehrerer Näpfe parallel zu überwachen.

Das große Streckungsverhältnis der zweiten Ausnehmungen führt zu mehreren Vorteilen. Beispielsweise können sich Zellprodukte, wie z.B. Proteine oder Antikörper, ohne oder bei langsamem Abtransport von Zellprodukten in den zweiten Ausnehmungen ansammeln. Dies kann zur Analyse dieser Zellprodukte und der Produktionsrate genutzt werden, z.B. indem Fluoreszenzmarker angebunden werden und die Intensität der Fluoreszenz ausgewertet wird. Werden Zellprodukte, z.B. Stoffwechselprodukte, schneller abtransportiert als sie von den Zellen produziert werden, können ideale Kultivierungsbedingungen geschaffen werden. Der Wechsel zwischen langsamem und schnellem Abtransport von Zellprodukten kann beispielsweise durch die Variation von Zu- und Ablaufraten des Nährmediums oder anderer Flüssigkeiten erreicht werden.

Die erste Ausnehmung kann in einem Kunststoff-, Metall-, Silizium- oder Glasteil ausgebildet werden, z.B. in Form einer Mikrotiterplatte, in Form einer Petrischale, als Dichtungsring oder als Rahmen, auch durch Verbindung verschiedener Materialien. Der Träger kann in einer Kultivierungsstation an vorhandene Zuläufe und Auslässe angeschlossen werden, insbesondere für Langzeitkultivierung. Zudem kann die Temperatur, die Gasatmosphäre, beispielsweise der CO₂-Gehalt, die Zusammensetzung der Flüssigkeiten, der Gehalt an Biomolekülen oder anderen Stoffen an Zuläufen eingestellt oder an Auslässen gemessen werden. An Auslässen können Sensoren angebracht sein, die pH-Wert, Fluoreszenz, Konzentrationen, Temperatur, Stoffwechselprodukte, von Zellen produzierte Biomoleküle usw. messen.

Durch eine oder mehrere Zuleitungen, z.B. in Form von Kapillaren, können verschiedene Flüssigkeiten in die erste Ausnehmung gegeben werden, z.B. Nährmedium, Pufferlösungen, Reagenzien, Färbemittel. Diese können Zellen enthalten. Durch einen oder mehrere Auslässe können die Flüssigkeiten wieder entnommen werden, um einen kontinuierlichen oder intervallhaften oder bedarfsgesteuerten Austausch zu gewährleisten. Zuleitungen können so angeordnet sein, dass die Zellen bei der Zuführung von Flüssigkeiten nicht beeinflusst werden. Bei der Verwendung einer Kapillare als Zuleitung kann Flüssigkeit insbesondere über einer zweiten Ausnehmung zugeführt werden, die keine Zellen enthält.

Aus der Flüssigkeit in der ersten Ausnehmung können Proben entnommen werden, beispielsweise um Produkte der Zellen (Proteine, Antikörper, Stoffwechselprodukte) in zeitlichen Abständen oder kontinuierlich zu detektieren, um die Konzentration von zugegebenen Reagenzien zu kontrollieren oder zur Analyse von Exosomen oder extrazellulären Vesikeln (EV), die von Zellen produziert werden, insbesondere die Beladung mit Art und Menge der Proteine und Nukleinsäuren, um Kenngrößen wie Heterogenität der EV in ihren biologischen und physikalischen Eigenschaften (Größe, Zusammensetzung in Art und Menge, Dichte, Brechungsindex) zu bestimmen.

Werden mehrere Einlässe verwendet, kann ein Teil davon zum Hinzufügen von nicht zellhaltigen Flüssigkeiten wie Medium, Wasser, etc. verwendet werden und ein anderer Teil zum Hinzufügen von zellhaltigen Flüssigkeiten.

Bei der Zufuhr von Flüssigkeit in die erste Ausnehmung oder die zweiten Ausnehmungen kann beispielsweise eine Flüssigkeit mit geringerer Dichte als die zellhaltige Flüssigkeit, die bevorzugt nicht mischbar mit der zellhaltigen Flüssigkeit ist, als temporärer Verdunstungsschutz dienen. Ist der erste Dickenabschnitt klein genug, können Zellen durch die Flüssigkeit im ersten Dickenabschnitt hindurch in die zweiten Ausnehmungen eingetragen werden. Parallel zum Eintrag der zellhaltigen Flüssigkeit kann eine zweite Zuleitung genutzt werden, um Verdunstung auszugleichen. Optional kann die abgegebene Flüssigkeit kontinuierlich in ihrer Zusammensetzung verändert werden, um das die Zellen umgebende Medium über einen Zeitraum hinweg auszutauschen.

Die Träger in verschiedenen Ausführungsformen sind so eingerichtet, dass Zellen in den zweiten Ausnehmungen kultiviert werden können. Zusätzlich können Verfahren zur Analyse der Zellen durchgeführt werden. Insbesondere können Zellen durch ein Färbemittel angefärbt werden. Dadurch, dass ein Austausch der Flüssigkeiten in der Umgebung der Zellen gewährleistet ist, kann das Färbemittel anschließend ausgewaschen werden, ohne die Zellen zu entnehmen oder anders zu beeinflussen.

Verschiedene Verfahren zur Zellkultivierung können angewendet werden. Insbesondere können Zellen in die zweiten Ausnehmungen mit sehr wenig Flüssigkeit gedruckt werden und anschließend Medium zugeführt werden. Alternativ kann erst Medium zugeführt werden und anschließend zellhaltige Flüssigkeit. Alternativ kann zellhaltiges Medium in einem Schritt zugeführt werden. Um sicherzustellen, dass die Zellen sich in den zweiten Ausnehmungen absetzen, kann zwischen den Verfahrensschritten gewartet werden. Zusätzlich kann das Absetzen der Zellen durch Zentrifugieren oder Agitieren, insbesondere Schütteln bzw. Vibrieren des Trägers unterstützt werden. Dadurch können zusätzlich Gasblasen in den zweiten Ausnehmungen entfernt werden. Anschließend kann zellhaltiges Medium entnommen werden, bis Zellen im Wesentlichen nur innerhalb der zweiten Ausnehmungen vorliegen.

Die Erfindung wird nun genauer anhand von Beispielen mit Bezug auf die Figuren beschrieben, die schematisch in
- Fig. 1 eine Ausführungsform der Erfindung,
- Fig. 2 eine Ausführungsform der Erfindung mit Zuleitung von der zweiten Oberfläche des Trägers,
- Fig. 3 eine Ausführungsform der Erfindung mit Zuleitung und Auslass von der zweiten Oberfläche des Trägers,
- Fig. 4 Ausführungsformen der Erfindung in Aufsicht,
- Fig. 5 eine Ausführungsform der Erfindung mit Zuleitung und Auslass,
- Fig. 6 eine weitere Ausführungsform der Erfindung mit Zuleitung und Auslass,
- Fig. 7 eine weitere Ausführungsform der Erfindung,
- Fig. 8 eine weitere Ausführungsform der Erfindung,
- Fig. 9 eine weitere Ausführungsform der Erfindung,
- Fig. 10 eine weitere Ausführungsform
zeigen.

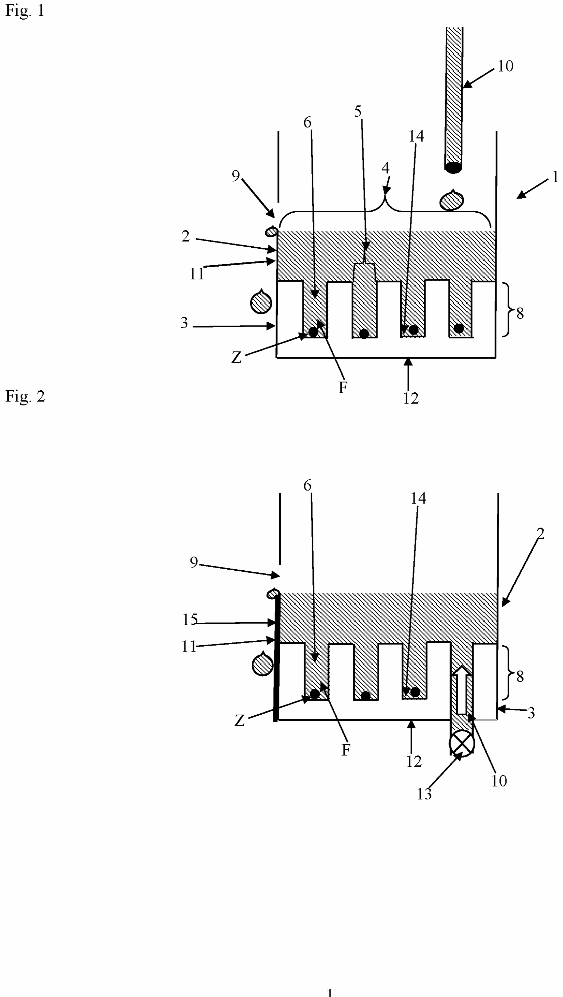

Die Figur 1 zeigt einen Träger 1 im Querschnitt, der aus einem ersten Teilträger 2 und einem daran angrenzenden zweiten Teilträger 3 besteht, wobei im ersten Teilträger 2 eine erste Ausnehmung 4 gebildet ist, die die Querschnittsöffnungen 5 der angrenzenden zweiten Ausnehmungen 6 überdeckt. Daher stehen die Innenvolumina der zweiten Ausnehmungen 6 entlang ihrer Querschnittsöffnungen 5 mit dem Innenvolumen der ersten Ausnehmung 4 in Kontakt, der einen Stoffaustausch zwischen diesen erlaubt, insbesondere durch Diffusion. Die zweiten Ausnehmungen 6 erstrecken sich über einen zweiten Dickenabschnitt 8 des Trägers 1, hier über einen zweiten Dickenabschnitt 8 im zweiten Teilträger 3. Im ersten Teilträger 2 erstreckt sich die erste Ausnehmung 4 über den ersten Dickenabschnitt 7, der dabei gleich der Dicke des ersten Teilträgers 2 ist. Der Träger 1 weist in seinem ersten Dickenabschnitt, hier gleich dem ersten Teilträger 2, einen Auslass 9 auf, durch den Flüssigkeit F, deren Pegel den Querschnitt des Auslasses 9 erreicht, austreten kann. Eine Zuleitung 10 mündet in einem Abstand oberhalb der Ebene, über die sich der Auslass 9 erstreckt. Die Zuleitung 10 ist nicht notwendigerweise mit dem Träger verbunden, sondern kann unabhängig vom Träger geführt sein, z.B. mittels einer Verfahreinrichtung (nicht dargestellt).

Das große Streckungsverhältnis der zweiten Ausnehmungen 6 erlaubt eine Bewegung der Flüssigkeit in der ersten Ausnehmung 4, ohne Zellen Z, die sich am Boden 14 oder im Volumen der zweiten Ausnehmung 6 befinden, aus den zweiten Ausnehmungen heraus zu spülen bzw. wobei eine Beeinflussung der Zellen durch Turbulenzen minimiert wird.

Die Figur 2 zeigt einen Träger 1 im Querschnitt, der wie der in Figur 1 gezeigt Träger 1 aus einem ersten Teilträger 2 mit einer ersten Ausnehmung 4 und einem daran angebrachten zweiten Teilträger 3 besteht, in dem sich zweite Ausnehmungen 6 angrenzend an die ersten Ausnehmung 4 über den zweiten Dickenabschnitt 8 erstrecken. Ein Auslass 9 ist im ersten Teilträger 2 gebildet. Der in den Figuren 1 und 2 dargestellte Auslass 9 erstreckt sich entlang einer äußeren Stirnfläche 11 des Trägers 1. Eine Zuleitung 10 erstreckt sich von der zweiten Oberfläche 12 des Träger 1 bzw. dessen zweiten Teilträgers 3 in die erste Ausnehmung 4. Wie bevorzugt weist die Zuleitung 10 ein gesteuertes Ventil 13 auf, um den Zulauf von Flüssigkeit F in die erste Ausnehmung 4 zu steuern.

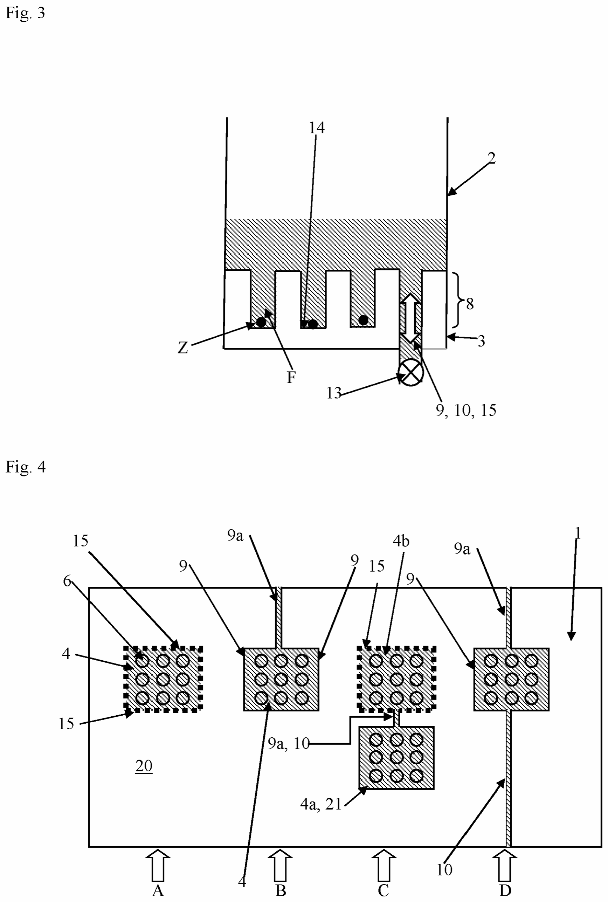

Die Figur 3 zeigt eine Ausführungsform des Trägers 1, bei der sich eine Verbindungsleitung 15, die einen Auslass 9 und bei entgegengesetzter Durchströmung eine Zuleitung 10 bildet, durch den Träger 1 von der ersten Ausnehmung 4 auf dessen zweite Oberfläche 12 erstreckt. Eine solche Verbindungsleitung 15, die sowohl eine Zuleitung 10 als auch einen Auslass 9 bildet, weist bevorzugt ein gesteuertes Ventil 13 auf, das weiter bevorzugt zwei oder mehr angeschlossene Leitungen aufweist, von denen z.B. eine mit einem Vorratsbehälter für Medium verbunden ist und eine weitere mit einem Auffangbehälter für aus der ersten Ausnehmung 4 abgeführte Flüssigkeit F. Diese Ausführungsform kann auch mit einem Deckel, der die erste Ausnehmung überdeckt, versehen werden. Bevorzugt weist der Deckel dann ein Ventil auf, mit dem die Zusammensetzung der Gasatmosphäre gesteuert werden kann.

Die Figur 4 zeigt Ausführungsformen der Erfindung in einem einstückigen Träger 1 in Aufsicht auf die erste Oberfläche 20 des Trägers 1 und auf die Querschnittsöffnung jeweils einer ersten Ausnehmung 4. Die erste Ausnehmung 4, hier rechteckig, erstreckt sich über die Querschnittsöffnungen der angrenzenden zweiten Ausnehmungen 6, die als Anordnung von 3 x 3 zweiten Ausnehmungen 6 je erster Ausnehmung 4 ausgebildet sind.

In einer Ausführungsform A von Figur 4 ist die erste Ausnehmung 4 von durch den Träger 1 hindurchgehenden Verbindungsleitungen 15 umgeben, die mit einem Auslass 9 verbunden sind oder den Auslass 9 bilden. Die Verbindungsleitungen 15 erstrecken sich von der ersten Ausnehmung durch den Träger 1 bis auf dessen der Querschnittsöffnung der ersten Ausnehmung 4 gegenüberliegende zweite Oberfläche 12. Die durch den Träger 1 hindurchgehenden Verbindungsleitungen 15 führen aus der ersten Ausnehmung austretende Flüssigkeit gezielt auf die gegenüberliegende zweite Oberfläche 12 des Trägers 1, so dass die Flüssigkeit dort gezielt abgenommen werden kann und auch eine weitere erste Ausnehmung 4, die in demselben Träger 1 gebildet ist, nicht kontaminiert.

In einer Ausführungsform B von Figur 4 ist eine erste Ausnehmung 4 mit einem Auslass 9 verbunden, der im Träger 1 in Form eines Grabens ausgebildet ist, dessen Querschnitt sich in den Träger 1 erstreckt. Der Auslass 9 ist am Umfang des Trägers 1 mit einer Verbindungsleitung 15 verbunden, die sich entlang der umfänglichen Stirnwand des Trägers 1 auf dessen zweite Oberfläche 12 erstreckt.

In einer Ausführungsform C von Figur 4 ist eine erste Ausnehmung 4a von einem umfänglichen Auslass 9 eingefasst, der durch einen Leitungsabschnitt 9a einen Zulauf in eine weitere erste Ausnehmung 4b bildet, so dass Flüssigkeit F durch den Auslass 9 und dessen Leitungsabschnitt 9a aus einer ersten Ausnehmung 4a in eine weitere erste Ausnehmung 4b fließen kann. Die erste Ausnehmung 4b ist von Verbindungsleitungen 15 eingefasst, durch die Flüssigkeit F, die aus dieser ersten Ausnehmung 4b austritt, auf die gegenüberliegende zweite Oberfläche 12 des Trägers 1 geleitet wird. Diese Ausführungsform zeigt auch als Beispiel für eine Vorratsausnehmung 21 eine erste Ausnehmung 4a, die mit einer Zuleitung 10 mit einer weiteren ersten Ausnehmung 4b verbunden ist.

In einer Ausführungsform C von Figur 4 können in den zweiten Ausnehmungen 6, die einer ersten Ausnehmung 4 zugeordnet sind, Zellen kultiviert werden, die bestimmte Biomoleküle, z.B. Zytokine, Antikörper, Proteine, herstellen. Durch den Leitungsabschnitt 9a können diese Moleküle in eine andere erste Ausnehmung 4 transportiert werden und dort detektiert werden oder auf andere Zellen einwirken.

In einer Ausführungsform D von Figur 4 ist wie auch mit Bezug auf die Ausführungsform B beschrieben, eine erste Ausnehmung 4 mit einem Auslass 9 verbunden, der im Träger 1 in Form eines Grabens ausgebildet ist, dessen Querschnitt sich in den Träger 1 erstreckt. Der Auslass 9 ist am Umfang des Trägers 1 mit einer Verbindungsleitung 15 verbunden, die sich entlang der umfänglichen Stirnwand 11 des Trägers 1 auf dessen zweite Oberfläche 12 erstreckt. Auf der dem Auslass 9 gegenüberliegenden Wand der ersten Ausnehmung 4 ist eine Zuleitung 10 im Träger 1 eingeformt, ebenfalls in Form eines Grabens.

Der Träger 1 kann eine oder mehrere erste Ausnehmungen 4 gleicher oder unterschiedlicher Form z.B. der Fig. 4A bis 4D aufweisen. Bei mehreren ersten Ausnehmungen 4 können in verschiedenen ersten Ausnehmungen 4 verschiedene Flüssigkeiten oder gleiche Flüssigkeiten in verschiedenen Konzentrationen oder Kombination davon enthalten sein. In den zu verschiedenen ersten Ausnehmungen 4 gehörenden zweiten Ausnehmungen 6 können unterschiedliche Zelltypen enthalten sein.

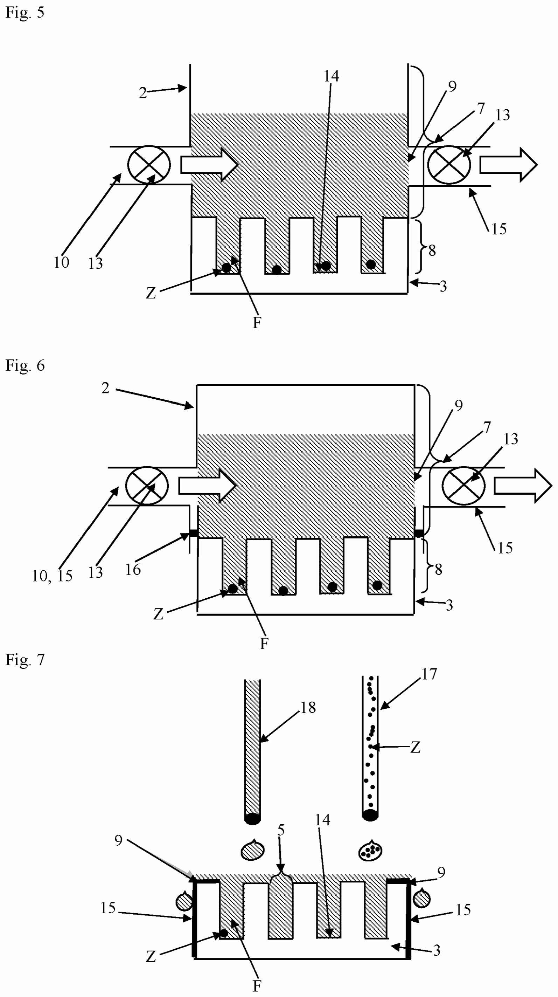

Die Figur 5 zeigt eine Ausführungsform, bei der die Wand, die sich über den ersten Dickenabschnitt 7 erstreckt und eine erste Ausnehmung 4 aufweist bzw. diese umfasst, von einem ersten Teilträger 2 gebildet sein, der an einen zweiten Teilträger 3 angrenzt, in dem eine Anordnung von zweiten Ausnehmungen 4 gebildet ist. In dem ersten Teilträger 2 kann eine Zuleitung 10 münden, die bevorzugt mit einer Verbindungsleitung 15 verbunden ist, und, bevorzugt der Zuleitung 10 gegenüber, ein Auslass 9 gebildet sein, der bevorzugt mit einer Verbindungsleitung 15 verbunden ist. Die hier gezeigte Zuleitung 10 und die Verbindungsleitung 15 sind mit jeweils einem gesteuerten Ventil 13 versehen, das eine Steuerung von Zufuhr und Abführung von Flüssigkeit F in bzw. aus der ersten Ausnehmung 4 erlaubt.

Wie in Figur 6 gezeigt, kann der erste Teilträger 2 zum zweiten Teilträger 3 verschieblich sein, z.B. dadurch, dass sich der erste Teilträger 2 und der zweite Teilträger 3 in ihren Randbereichen verschieblich und flüssigkeitsdicht überlappen. Zwischen dem ersten Teilträger 2 und dem zweiten Teilträger 3 kann eine Dichtung 16 angeordnet sein, die z.B. ein Grat auf dem aus Kunststoff bestehenden ersten Teilträger 2 oder zweiten Teilträger 3 oder ein Gummiring ist. Weiter zeigt Figur 6, dass der erste Teilträger 2 die erste Ausnehmung 4 überdecken kann. Der überdeckende Teil des ersten Teilträgers 2 kann als Deckel ausgeführt sein, der wiederverwertbar ist.

Die Figur 7 zeigt ein Verfahren zum separaten Einbringen von zellhaltiger Flüssigkeit mittels einer ersten Dosiereinrichtung 17 und, vorher, anschließend oder gleichzeitig, Einbringen von zellfreiem Medium mittels einer zweiten Dosiereinrichtung 18, in Ausnehmungen 4, 6 des Trägers 1.

Die Figur 7 zeigt einen Träger 1, dessen Ausnehmungen aus einer Anordnung von zweiten Ausnehmungen 6 besteht und eine diese überdeckende Schicht aus Flüssigkeit, die den ersten Dickenabschnitt 7 bildet, durch die Oberflächenspannung gehalten wird. Dazu ist bevorzugt, dass die Umfassung der Anordnung von zweiten Ausnehmungen 6 hydrophob ist, z.B. eine hydrophobe Beschichtung aufweist. Der Träger 1 weist als Auslass 9 z.B. einen hydrophilen Bereich auf, der an eine Verbindungsleitung 15 angrenzt, die sich entlang der Stirnfläche 11 des Trägers 1 zu dessen zweiter Oberfläche 12 erstreckt, die den Querschnittsöffnungen der Ausnehmungen 6 gegenüberliegt. Die hydrophobe Beschichtung, die die zweiten Ausnehmungen 6 umfasst, ist bevorzugt so ausgelegt, dass auf den Träger gegebene Tropfen einer Flüssigkeit zu einer zusammenhängenden Schicht zusammenlaufen und erst dann am Auslass 9 ablaufen. Alternativ kann der Auslass 9 durch Mikrostrukturen in der Oberfläche des Trägers 1 gebildet werden.

Im Verfahren kann das Ablaufen der Flüssigkeit über den Auslass 9 durch Kippen des Trägers 1 oder durch Inkontaktbringen der Flüssigkeit mit einer Vorrichtung mit Kapillarwirkung gesteuert werden.

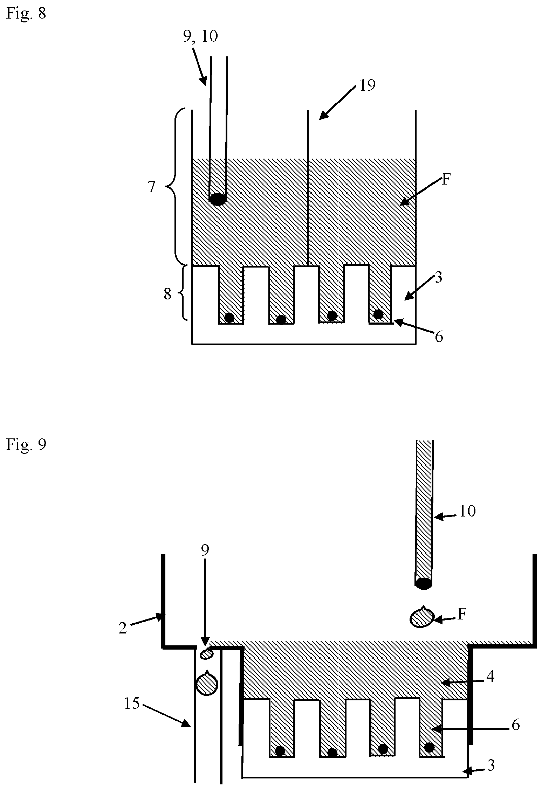

Die Figur 8 zeigt einen Träger 1, in dessen ersten Teilträger 2 optional kein Auslass 9 angebracht ist, sondern der Einlass 10 zugleich als Dosiereinrichtung 17 und als Auslass 9 dient. Alternativ können Einlass und Auslass als zwei Dosiereinrichtungen 17, 18 realisiert sein. Die erste Ausnehmung ist durch eine Membran 19, die bevorzugt semipermeabel ist, senkrecht zur Querschnittsöffnung der ersten Ausnehmung unterteilt, wodurch das gemeinsame Flüssigkeitsvolumen der ersten Ausnehmung 4 oberhalb der zweiten Ausnehmungen 6 unterteilt ist. Die Membran 19 kann in analoger Weise auch mit anderen Ausführungsformen kombiniert werden und dort die erste Ausnehmung 4 bzw. den ersten Dickenabschnitt 7 unterteilen.

Die Figur 9 zeigt eine Ausführungsform, bei der der Träger 1 aus einem ersten Teilträger 2, bevorzugt aus Kunststoff, und einem zweiten Teilträger 3, bevorzugt aus Glas, besteht, wobei der erste Teilträger 2 entlang des Umfangs des zweiten Teilträgers 3 mit diesem verbunden ist. Eine erste Ausnehmung 4 erstreckt sich über zweite Ausnehmungen 6, die im zweiten Teilträger 3 ausgebildet sind. Der erste Teilträger 2 weist in einem Abstand zu dem Bereich seiner ersten Ausnehmung 4, bevorzugt in einem Abstand zu dem Bereich, der vom zweiten Teilträger 3 überdeckt wird, einen Auslass 9 auf. Eine Zuleitung 10 mündet in einem Abstand oberhalb der ersten Ausnehmung 4, um Tropfen von Flüssigkeit F in die erste Ausnehmung 4 zu dosieren. Der erste Teilträger 2 weist bevorzugt gegenüber dem zweiten Teilträger 3 eine Querschnittsöffnung auf, in oder gegen die die Zuleitung 10 gerichtet ist. Bevorzugt ist eine Verbindungsleitung 15 am Auslass 9 angeschlossen.

Im Verfahren sind die zweiten Ausnehmungen 6 und die erste Ausnehmung 4 mit Flüssigkeit F, die insbesondere Medium ist, gefüllt, und stehen durch die Durchgangsbohrungen 6c miteinander in Kontakt. Insbesondere in einer Ausführungsform, in der eine Zuleitung 10 unterhalb der zweiten Ausnehmungen 6, bzw. unterhalb des zweiten Teilträgers 3 im ersten Teilträger 2 mündet, kann zusätzlich oder alternativ zu Flüssigkeit F ein Gas, z.B. Inertgas oder Luft, z.B. Luft oder Sauerstoff für die Zellkultivierung, optional Luft oder O₂ mit 5 % CO₂, durch die Zuleitung 10 eingeführt werden. Das Gas kann blasenfrei zugeführt werden, indem der Volumenstrom des Gases hinreichend gering ist. Alternativ kann das Gas mit einem Volumenstrom zugeführt werden, der zur Bildung von Blasen ausreicht, wobei Flüssigkeit F in den zweiten Ausnehmungen bewegt wird und optional Zellen Z mittels der Gasblasen G flotiert werden. Gasblasen können alternativ durch Kavitation, die mit Laserstrahlung ausgelöst wird, erzeugt werden.

Durch Druckveränderungen können Zellen Z von den Durchgangsbohrungen 6c wegbewegt werden, um den Austausch der Flüssigkeiten zu unterstützen.

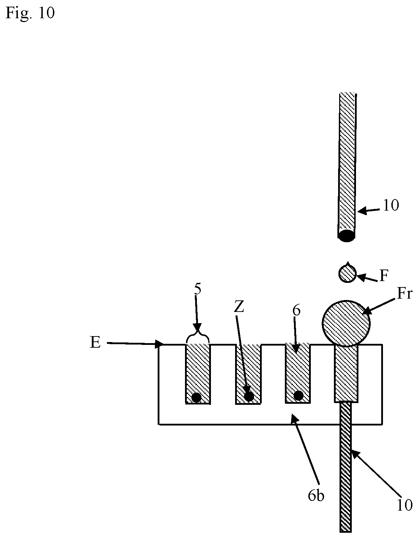

Die Fig. 10 zeigt schematisch eine Ausführungsform, in der der Träger 1 aus einem zweiten Teilträger 3 mit zweiten Ausnehmungen 6 besteht, deren Querschnittsöffnungen 5 und Bereiche der Oberfläche des Trägers 1, 3 zwischen diesen Querschnittsöffnungen 5 in einer gemeinsamen Ebene E liegen. Insbesondere, wenn diese Oberflächenbereiche zwischen den Querschnittsöffnungen 5 eine Oberflächenenergie aufweisen, die eine Benetzung durch die Flüssigkeit F nicht oder nur wenig zulässt, z.B. Oberflächenbereiche aus Kunststoff oder funktionalisiertem, beschichteten oder hydrophob strukturiertem Glas, kann eine sich über die Ebene E bewegende Flüssigkeit F, z.B. in Form eines rollenden Tropfens Fr, verbrauchtes Medium aus den zweiten Ausnehmungen 6 aufnehmen und zumindest teilweise ersetzen. Ein rollender Tropfen Fr kann z.B. durch Aufbringen von Flüssigkeit F in Form mehrerer Tropfen auf die Ebene E oder in zweite Ausnehmungen 6, z.B. durch eine in eine zweite Ausnehmung 6 mündende Zuleitung 10, wie z.B. in Fig. 2 gezeigt, eingebracht werden. Über die Ebene E ragende Flüssigkeit F, insbesondere in Form eines rollenden Tropfens Fr, kann durch Neigen des Trägers 1 gegen die Horizontale, durch Beschleunigen und/oder Vibrieren des Trägers, durch Beaufschlagen mit einem Gasstrom angetrieben werden.

Generell kann in Ausführungsformen, in denen der Träger aus einem zweiten Teilträger 3 mit zweiten Ausnehmungen 6 darin besteht, der zweite Teilträger als Träger 3 mit Ausnehmungen 6 darin bezeichnet werden.

### Bezugszeichen:

| | | | |
|---|---|---|---|
| 1 | Träger | 16 | Dichtung |
| 2 | erster Teilträger | 17 | erste Dosiereinrichtung |
| 3 | zweiter Teilträger | 18 | zweite Dosiereinrichtung |
| 4, 4a, 4b | erste Ausnehmung | 19 | Membran |
| 5 | Querschnittsöffnung einer zweiten | 20 | erste Oberfläche des Trägers |
| | Ausnehmung | 21 | Vorratsausnehmung |
| 6 | zweite Ausnehmung | 22 | Material |
| 6b | Boden einer zweiten Ausnehmung | 23 | Übergangsleitung |
| 6c | Durchgangsbohrung | 24 | Bodenausnehmung in zweitem Teilträger |
| 7 | erster Dickenabschnitt | | |
| 8 | zweiter Dickenabschnitt | 25 | Abstandshalter |
| 9 | Auslass | E | Ebene |
| 9a | Leitungsabschnitt des Auslasses | F | Flüssigkeit |
| 10 | Zuleitung | Fr | rollender Tropfen |
| 11 | Stirnwand, Stirnfläche | Z | Zelle |
| 12 | zweite Oberfläche des Trägers | G | Gasblase |
| 13 | gesteuertes Ventil | | |
| 14 | Boden der zweiten Ausnehmung | | |
| 15 | Verbindungsleitung | | |

## Patentansprüche

1. Träger (1) zur Kultivierung von Zellen (Z), der zumindest eine erste Ausnehmung (4, 4a, 4b) aufweist, die sich über einen ersten Dickenabschnitt (7) von einer ersten Oberfläche (20) des Trägers (1) bis an einen zweiten Dickenabschnitt (8) erstreckt, der an den ersten Dickenabschnitt (7) angrenzt und in dem sich von der ersten Ausnehmung (4, 4a, 4b) eine Anordnung von mindestens 90 zweiten Ausnehmungen (6) erstreckt, wobei die Querschnittsöffnung der ersten Ausnehmung (4, 4a, 4b) in der Ebene der ersten Oberfläche (20) des Trägers (1) liegt, **dadurch gekennzeichnet, dass** die zweiten Ausnehmungen (6) zur Aufnahme von Zellen geeignete Näpfe bilden, die ein Streckungsverhältnis von Tiefe zu Durchmesser von zumindest 1:1 und einen Durchmesser von maximal 1 mm aufweisen und dadurch, dass der Träger (1) einen Auslass (9) aufweist, dessen Querschnitt im oder angrenzend an den ersten Dickenabschnitt (7) mündet und dadurch, dass in der ersten Oberfläche (20) oder im ersten Dickenabschnitt (7) des Trägers (1) eine Zuleitung (10) gebildet ist, die in die erste Ausnehmung (4,4a, 4b) mündet.

2. Träger (1) nach Anspruch 1, **dadurch gekennzeichnet, dass** der Auslass (9) einen in den Träger (1) ragenden Querschnitt aufweist, der zur Ebene der ersten Oberfläche (20) des Trägers (1) offen ist.

3. Träger (1) nach einem der voranstehenden Ansprüche, **gekennzeichnet durch** eine Verbindungsleitung (15), die mit dem Auslass (9) verbunden ist und sich bis auf die der ersten Oberfläche (20) des Trägers (1) gegenüberliegende zweite Oberfläche (12) erstreckt.

4. Träger (1) nach Anspruch 3, **dadurch gekennzeichnet, dass** sich die Verbindungsleitung (15) entlang einer Stirnwand (11) des Trägers (1) oder durch den Träger (1) erstreckt.

5. Träger (1) nach einem der Ansprüche 3 bis 4, **dadurch gekennzeichnet, dass** der Auslass (9) und die daran anschließende Verbindungsleitung (15) eine hydrophile Oberfläche aufweisen, und dass Bereiche der ersten Oberfläche (20) und/oder Bereiche der zweiten Oberfläche (12) des Trägers (1) hydrophob sind.

6. Träger (1) nach einem der voranstehenden Ansprüche, **dadurch gekennzeichnet, dass** er einstückig aus Glas besteht.

7. Träger (1) nach einem der voranstehenden Ansprüche, **dadurch gekennzeichnet, dass** er aus einem zweiten Teilträger (3) besteht, der sich über den zweiten Dickenabschnitt (8) erstreckt und aus einem ersten Teilträger (2), der sich über den ersten Dickenabschnitt (7) erstreckt, wobei der erste (2) und der zweite (3) Teilträger flüssigkeitsdicht zueinander angeordnet sind.

8. Träger (1) nach einem der voranstehenden Ansprüche, **dadurch gekennzeichnet, dass** er aus einem zweiten Teilträger (3) besteht, der sich über den zweiten Dickenabschnitt (8) erstreckt und aus einem ersten Teilträger (2), der sich über den ersten Dickenabschnitt (7) erstreckt, wobei der erste (2) und der zweite (3) Teilträger flüssigkeitsdicht zueinander und aneinander verschieblich angeordnet sind.

9. Träger (1) nach einem der Ansprüche 7 bis 8, **dadurch gekennzeichnet, dass** der erste Teilträger (2) aus Glas, Kunststoff oder Silizium besteht.

10. Träger (1) nach einem der Ansprüche 7 bis 9, **dadurch gekennzeichnet, dass** der zweite Teilträger (3) aus Glas oder Silizium besteht.

11. Träger (1) nach einem der voranstehenden Ansprüche, **dadurch gekennzeichnet, dass** in dem Träger (1) zumindest zwei erste Ausnehmungen (4, 4a, 4b) gebildet sind, von denen jede durch umfänglich angeordnete Verbindungsleitung (15) umfasst ist, die sich durch den Träger (1) erstrecken.

12. Träger (1) nach einem der voranstehenden Ansprüche, **dadurch gekennzeichnet, dass** in dem Träger (1) zumindest zwei erste Ausnehmungen (4, 4a, 4b) gebildet sind, von denen zumindest eine von einem Auslass (9) in Form eines Grabens eingefasst ist, der in eine andere der ersten Ausnehmungen (4, 4a, 4b) mündet, die durch umfänglich angeordnete Verbindungsleitungen (15) umfasst ist, die sich durch den Träger (1) erstrecken.

13. Träger (1) nach einem der Ansprüche 11 bis 12, **dadurch gekennzeichnet, dass** in einem Auslass (9), in einer Verbindungsleitung (15) und/oder in einer Zuleitung (10) jeweils ein gesteuertes Ventil (13) angeordnet ist.

14. Verfahren zur Kultivierung von Zellen, **gekennzeichnet durch** Einbringen von Flüssigkeit (F), in der Zellen (Z) suspendiert sind, in zweite Ausnehmungen (6) eines Trägers (1) nach einem der voranstehenden Ansprüche, mit anschließendem Abführen von Flüssigkeit aus dem ersten Dickenabschnitt (4, 4a, 4b) entlang des Auslasses (9) und der Verbindungsleitung (15).

15. Verfahren nach Anspruch 14, **dadurch gekennzeichnet, dass** der erste Teilträger (2) nur durch die Oberfläche des zweiten Teilträgers (3), durch die die zweiten Ausnehmungen (6) beabstandet sind, gebildet wird und die an die Anordnung von zweiten Ausnehmungen (6) angrenzende erste Ausnehmung (4, 4a, 4b) allein durch eine durch Oberflächenspannung erzeugte Flüssigkeitsschicht gebildet wird.

16. Verfahren nach einem der Ansprüche 14 bis 15, **dadurch gekennzeichnet, dass** die Oberfläche des zweiten Teilträgers (3), durch die die zweiten Ausnehmungen (6) beabstandet sind, eine hydrophobe Oberfläche ist.

17. Verfahren nach einem der Ansprüche 14 bis 16, **dadurch gekennzeichnet, dass** der Träger (1) nur zweite Ausnehmungen (6) aufweist und Medium eine über die Ebene der Querschnittsöffnungen der zweiten Ausnehmungen (6) ragende Schicht bildet, die das Volumen der ersten Ausnehmung bildet.

18. Verfahren nach einem der Ansprüche 14 bis 17, **dadurch gekennzeichnet, dass** die im Träger (1, 3) Ausnehmungen (6) aufweist, deren Querschnittsöffnungen (5) durch Oberflächenbereiche des Trägers (1, 3) beabstandet sind, wobei diese Oberflächenbereiche und die Querschnittsöffnungen (5) der Ausnehmungen (6) in einer gemeinsamen Ebene (E) liegen, wobei Flüssigkeit (F) auf diese Ebene (E) aufgebracht wird und durch Neigen des Trägers (1, 3) gegen die Horizontale, Vibrieren des Trägers (1, 3) und/oder Beaufschlagen der Ebene (E) mit Druckgas die Flüssigkeit (F) entlang des Trägers (1, 3) bewegt wird.

19. Verfahren nach Anspruch 18, **dadurch gekennzeichnet, dass** die Flüssigkeit (F) mittels einer Zuleitung (10), die im Boden (6c) einer Ausnehmung (6) mündet, auf diese Ebene (E) aufgebracht wird.

## Claims

1. Carrier (1) for cultivating cells (Z), the carrier having at least one first recess (4, 4a, 4b) extending over a first thickness section (7) from a first surface (20) of the carrier (1) up to a second thickness section (8) which is adjacent to the first thickness section (7) and in which an arrangement of at least 90 second recesses (6) extends from the first recess (4, 4a, 4b), wherein the cross-sectional opening of the first recess (4, 4a, 4b) extends in the plane of the first surface (20) of the carrier (1), **characterized in that** the second recesses (6) form wells suitable for receiving cells, which wells have an aspect ratio of depth to diameter of at least 1:1 and a diameter of at most 1 mm, and **in that** the carrier (1) has an outlet (9) whose cross-section opens in or adjacent to the first thickness section (7), and **in that** that a supply conduit (10) is formed in the first surface (20) or in the first thickness section (7) of the carrier (1), which supply conduit (10) opens into the first recess (4, 4a, 4b).

2. Carrier (1) according to claim 1, **characterized in that** the outlet (9) has a cross-section projecting into the carrier (1), which cross-section is open to the plane of the first surface (20) of the carrier (1).

3. Carrier (1) according to one of the preceding claims, **characterized by** a connection conduit (15) which is connected to the outlet (9) and which extends to the second surface (12) that lies opposite to the first surface (20) of the carrier (1).

4. Carrier (1) according to claim 3, **characterized in that** the connection conduit (15) extends along an end wall (11) of the carrier (1) or through the carrier (1).

5. Carrier (1) according to one of claims 3 to 4, **characterized in that** the outlet (9) and the connection conduit (15) adjacent thereto have a hydrophilic surface, and **in that** regions of the first surface (20) and/or regions of the second surface (12) of the carrier (1) are hydrophobic.

6. Carrier (1) according to one of the preceding claims, **characterized in that** it is made single-pieced of glass.

7. Carrier (1) according to one of the preceding claims, **characterized in that** it consists of a second partial carrier (3) extending over the second thickness section (8) and of a first partial carrier (2) extending over the first thickness section (7), the first (2) and second (3) partial carriers being arranged in a liquid-tight manner relative to one another.

8. Carrier (1) according to one of the preceding claims, **characterized in that** it consists of a second partial carrier (3) extending over the second thickness section (8) and of a first partial carrier (2) extending over the first thickness section (7), the first (2) and the second (3) partial carriers being arranged in a liquid-tight manner to one another and displaceably against one another.

9. Carrier (1) according to one of the preceding claims, **characterized in that** the first partial carrier (2) consists of glass, plastic and/or silicon.

10. Carrier (1) according to one of claims 7 to 9, **characterized in that** the second partial carrier (3) consists of glass and/or silicon.

11. Carrier (1) according to one of the preceding claims, **characterized in that** in the carrier (1) at least two first recesses (4, 4a, 4b) are formed, each of which is enclosed by circumferentially arranged connection conduits (15) extending through the carrier (1).

12. Carrier (1) according to one of the preceding claims, **characterized in that** in the carrier (1) at least two first recesses (4, 4a, 4b) are formed, at least one of which is enclosed by an outlet (9) in the form of a trench that opens into another one of said first recesses (4, 4a, 4b), which is enclosed by circumferentially arranged connection conduits (15) extending through said carrier (1).

13. Carrier (1) according to one of the preceding claims, **characterized in that** a controlled valve (13) is arranged each in an outlet (9), in a connection conduit (15) and/or in a supply conduit (10).

14. Method for cultivating cells, **characterized by** introducing liquid (F) in which cells (Z) are suspended into second recesses (6) of a carrier (1) according to one of the preceding claims, with subsequent removal of liquid from the first thickness section (4, 4a, 4b) along the outlet (9) and the connection conduit (15).

15. Method according to claim 18, **characterized in that** the first partial carrier (2) is formed only by the surface of the second partial carrier (3) by which the second recesses (6) are spaced apart, and the first recess (4, 4a, 4b) adjacent to the arrangement of second recesses (6) is formed solely by a liquid layer generated by surface tension.

16. Method according to one of claims 14 to 15, **characterized in that** the surface of the second partial carrier (3) by which the second recesses (6) are spaced apart, is a hydrophobic surface.

17. Method according to one of claims 14 to 16, **characterized in that** the carrier (1) has only second recesses (6) and medium forms a layer projecting above the plane of the cross-sectional openings of the second recesses (6), which layer forms the volume of the first recess.

18. Method according to one of claims 14 to 17, **characterized in that** the carrier (1, 3) has recesses (6) whose cross-sectional openings (5) are spaced apart by surface regions of the carrier (1, 3), these surface regions and the cross-sectional openings (5) of the recesses (6) lying in a common plane (E), wherein liquid (F) is applied onto this plane (E) and the liquid (F) is moved along the carrier (1, 3) by tilting the carrier (1, 3) against the horizontal, by vibrating the carrier (1, 3), and/or by applying pressurized gas to the plane (E).

19. Method according to claim 18, **characterized in that** the liquid (F) is applied to this plane (E) by means of a supply conduit (10) opening in the bottom (6c) of a recess (6).

## Revendications

1. Support (1) pour la culture de cellules (Z), qui présente au moins un premier évidement (4, 4a, 4b) qui s'étend sur une première section d'épaisseur (7) depuis une première surface (20) du support (1) jusqu'à une deuxième section d'épaisseur (8) qui est adjacente à la première section d'épaisseur (7) et dans laquelle s'étend depuis le premier évidement (4, 4a, 4b) un ensemble d'au moins 90 deuxièmes évidements (6), l'ouverture en section transversale du premier évidement (4, 4a, 4b) étant située dans le plan de la première surface (20) du support (1), **caractérisé en ce que** les deuxièmes évidements (6) forment des cuvettes adaptées pour recevoir des cellules, qui ont un rapport d'étirement de la profondeur au diamètre d'au moins 1 :1 et un diamètre d'au maximum 1 mm, et **en ce que** le support (1) comporte une sortie (9) dont la section transversale débouche dans ou à proximité de la première section d'épaisseur (7), et **en ce qu'**un conduit d'alimentation (10) est formé dans la première surface (20) ou dans la première section d'épaisseur (7) du support (1) et débouche dans le premier évidement (4, 4a, 4b).

2. Support (1) selon la revendication 1, **caractérisé en ce que** la sortie (9) présente une section transversale faisant saillie dans le support (1) et ouverte vers le plan de la première surface (20) du support (1).

3. Support (1) selon l'une des revendications précédentes, **caractérisé par** un conduit de liaison (15) relié à la sortie (9) et s'étendant jusqu'à la deuxième surface (12) opposée à la première surface (20) du support (1).

4. Support (1) selon revendication 3, **caractérisé en ce que** le conduit de liaison (15) s'étend le long d'une paroi frontale (11) du support (1) ou à travers le support (1).

5. Support (1) selon l'une des revendications 3 à 4, **caractérisé en ce que** la sortie (9) et la conduite de liaison (15) qui s'y raccorde présentent une surface hydrophile, et **en ce que** des zones de la première surface (20) et/ou des zones de la deuxième surface (12) du support (1) sont hydrophobes.

6. Support (1) selon l'une des revendications précédentes, **caractérisé en ce qu'**il est constitué d'une seule pièce de verre.

7. Support (1) selon l'une des revendications précédentes, **caractérisé en ce qu'**il est constitué d'un deuxième support partiel (3) qui s'étend sur la deuxième section d'épaisseur (8) et d'un premier support partiel (2) qui s'étend sur la première section d'épaisseur (7), le premier (2) et le deuxième (3) supports partiels étant disposés de manière étanche aux liquides l'un par rapport à l'autre.

8. Support (1) selon l'une des revendications précédentes, **caractérisé en ce qu'**il est constitué d'un deuxième support partiel (3) qui s'étend sur la deuxième section d'épaisseur (8) et d'un premier support partiel (2) qui s'étend sur la première section d'épaisseur (7), le premier (2) et le deuxième (3) supports partiels étant disposés de manière étanche aux liquides l'un par rapport à l'autre et pouvant coulisser l'un par rapport à l'autre.

9. Support (1) selon l'une des revendications 7 à 8, **caractérisé en ce que** le premier support partiel (2) consiste en verre, en matière plastique ou en silicium.

10. Support (1) selon l'une des revendications 7 à 9, **caractérisé en ce que** le deuxième support partiel (3) consiste en verre ou en silicium.

11. Support (1) selon l'une des revendications précédentes, **caractérisé en ce qu'**au moins deux premiers évidements (4, 4a, 4b) sont formés dans le support (1), chacun d'entre eux étant entouré par des conduits de connexion (15) disposées circonférentiellement et s'étendant à travers le support (1).

12. Support (1) selon l'une des revendications précédentes, **caractérisé en ce que** dans le support (1) sont formés au moins deux premiers évidements (4, 4a, 4b) dont l'un au moins est bordé par une sortie (9) en forme de tranchée débouchant dans un autre des premiers évidements (4, 4a, 4b) compris par des conduits de liaison (15) disposés circonférentiellement et s'étendant à travers le support (1).

13. Support (1) selon l'une des revendications 11 à 12, **caractérisé en ce qu'**une vanne commandée (13) est disposée dans une sortie (9), dans un conduit de liaison (15) et/ou dans un conduit d'alimentation (10).

14. Procédé de culture de cellules, **caractérisé par** l'introduction de liquide (F), dans lequel des cellules (Z) sont en suspension, dans des deuxièmes évidements (6) d'un support (1) selon l'une des revendications précédentes, suivie de l'évacuation du liquide hors de la première section d'épaisseur (4, 4a, 4b) le long de la sortie (9) et du conduit de liaison (15).

15. Procédé selon la revendication 14, **caractérisé en ce que** le premier support partiel (2) est formé uniquement par la surface du deuxième support partiel (3) par laquelle les deuxièmes évidements (6) sont espacés, et la première cavité (4, 4a, 4b) adjacente à l'ensemble de deuxièmes évidements (6) est formée uniquement par une couche de liquide générée par la tension superficielle.

16. Procédé selon l'une des revendications 14 à 15, **caractérisé en ce que** la surface du deuxième support partiel (3), par laquelle les deuxièmes évidements (6) sont espacés, est une surface hydrophobe.

17. Procédé selon l'une des revendications 14 à 16, **caractérisé en ce que** le support (1) ne comporte que des deuxièmes évidements (6) et **en ce que** un medium forme une couche faisant saillie au-dessus du plan des ouvertures de section des deuxièmes évidements (6) et constituant le volume du premier évidement.

18. Procédé selon l'une des revendications 14 à 17, **caractérisé en ce que** le support (1, 3) présente des évidements (6) dont les ouvertures de section (5) sont espacées par des zones de surface du support (1, 3), ces zones de surface et les ouvertures de section (5) des évidements (6) étant situées dans un plan commun (E), un liquide (F) étant appliqué sur ce plan (E) et le liquide (F) étant déplacé le long du support (1, 3) par inclinaison du support (1, 3) par rapport à l'horizontale, par vibration du support (1, 3) et/ou par sollicitation du plan (E) par un gaz sous pression.

19. Procédé selon revendication 18, **caractérisé en ce que** le liquide (F) est appliqué sur ce plan (E) au moyen d'une conduite d'alimentation (10) qui débouche dans le fond (6c) d'un évidement (6).
